(19) Europäisches Patentamt / European Patent Office / Office européen des brevets

(11) **EP 3 426 341 B1**

(12) **EUROPEAN PATENT SPECIFICATION**

(45) Date of publication and mention
of the grant of the patent:
**09.06.2021 Bulletin 2021/23**

(51) Int Cl.:
***A61N 5/06*** (2006.01)

(21) Application number: **16893031.1**

(86) International application number:
**PCT/CN2016/075918**

(22) Date of filing: **09.03.2016**

(87) International publication number:
**WO 2017/152379 (14.09.2017 Gazette 2017/37)**

(54) **FAR INFRARED RADIATION HEAT PATCH**

WÄRMEPATCH MIT FERNINFRAROTSTRAHLUNG

PATCH DIFFUSEURS DE CHALEUR À RAYONNEMENT INFRAROUGE LOINTAIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
MA MD**

(43) Date of publication of application:
**16.01.2019 Bulletin 2019/03**

(73) Proprietor: **Johnson & Johnson Consumer Inc.
Skillman, NJ 08558 (US)**

(72) Inventors:
• **SHENG, Ling
Shanghai 201100 (CN)**
• **WU, Ace
Shanghai 200030 (CN)**
• **PAN, Robert
Cincinnati, OH 45242 (US)**

(74) Representative: **Kirsch, Susan Edith
Carpmaels & Ransford LLP
One Southampton Row
London WC1B 5HA (GB)**

(56) References cited:
**EP-A1- 2 908 807          EP-A2- 0 963 138
CN-A- 1 161 870          CN-A- 1 161 870
CN-Y- 2 732 088          CN-Y- 201 085 373
JP-A- H0 197 476          JP-A- H1 043 319
JP-A- H1 043 319          US-A- 6 108 581
US-A1- 2012 191 164          US-A1- 2015 335 742**

**Description**

BACKGROUND OF THE INVENTION

Field of the Invention

The present invention relates to therapeutic treatment devices (e.g., patches). More particularly, the present invention relates to therapeutic treatment devices using far infrared radiation (FIR).

Related Background Art

People suffering from chronic pain typically take pain medicines to relieve the pain. However, the relief from pain is only temporary. Pain medicines may also have undesirable side effects.

Some people seek alternative treatments such as traditional Chinese medicine (TCM) and/or physical treatment(s). One alternative treatment option is far infrared radiation treatment. FIR has been used to provide instant pain relief and to activate the immune system to provide long term restoration to damaged muscles. FIR covers the electromagnetic spectrum with wavelengths between 3 and 100 $\mu$m. Of particular interest are wavelengths between about 3 to about 25 $\mu$m, more specifically from about 5 to about 25 $\mu$m.

The present invention helps prevent and relive pain, especially chronic muscle pain. It provides pain relief without having to ingest drug medications and without the side effects of drug medications. The present invention utilizes FIR to provide pain relief in the form of a heat patch applied to the skin of a subject. The FIR device is designed for people to wear anytime and anywhere. FIR patches provide low energy to the skin of a subject and have pain relieving effects. JPH1043319A relates to an infrared thermal pad for a car seat. US2012/191164A1 relates to a method and apparatus for hearing via infrared radiation. EP0963138A2 relates to a far infrared ray diffusion mat. EP2908807A1 relates to a skin patch for absorbing toxins from the body. US2015/335742A1 relates to articles, compositions, systems, and methods of using and preparing bioceramic compositions. CN1161870A relates to a kind of far infrared radiation plaster.

SUMMARY OF THE INVENTION

The present disclosure is directed to a thermal treatment device comprising a far infrared ray material, a radiation reflection layer, and a heat energy source, wherein the radiation reflection layer is positioned between the far infrared ray material and the heat energy source.

The invention is as defined in the appended claims. Aspects, examples and embodiments of the present disclosure which do not fall under the scope of the claims, in particular methods, do not form part of the invention and are presented for illustration purposes only.

The present invention includes a thermal treatment device comprising a far infrared ray material, a radiation reflection layer, and a heat energy source, wherein the radiation reflection layer is positioned between the far infrared ray material and the heat energy source, and wherein the device has an emissivity of at least 0.75 at about 40°C to about 45°C.

The present invention also includes a process for making a thermal treatment device, comprising the steps of providing a far infrared ray material and a heat energy source, and placing a radiation reflective layer between the far infrared material and the heat energy source.

The present disclosure further includes a method of treating body pain, comprising the step of applying a thermal treatment device to a subject, wherein the device comprises far infrared ray material, a radiation reflection layer, and a heat energy source, wherein the radiation reflection layer is positioned between the far infrared ray material and the heat energy source.

BRIEF DESCRIPTION OF THE DRAWINGS

The detailed description will be better understood in conjunction with the accompanying drawings, wherein like reference characters represent like elements, as follows:

FIG. 1 depicts a FIR measuring device;
FIG. 2A, 2B and 2C illustrate FIR heat patch devices of the prior art;
FIG. 3 illustrates one embodiment of the inventive FIR Heat Patch Device; and
FIG. 4 illustrates an alternative embodiment of the inventive FIR Patch Device.

## DETAILED DESCRIPTION OF THE INVENTION

As used herein, "emissivity" refers to the measure of an object's ability to emit infrared energy. The energy emitted provides an indication of the temperature of the object. Emissivity can have a value from 0 (e.g., a shiny mirror) to 1.0 (e.g., a blackbody).

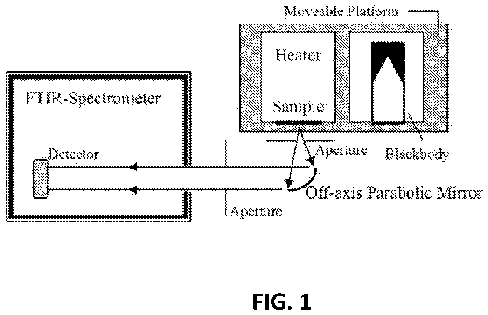

The emissivity is determined using a FIR measuring device like the one depicted in FIG. 1. Measurements were performed with an FTIR system, which consists of a heating system and a detecting system. The heating system includes a sample heater and a type IR563/301 blackbody with a temperature stability up to 0.2 K per 24-h period. The heating system is fixed on a moveable platform, which is enabled to switch between the heated sample and the blackbody for radiation detection with the same optical path length. The detecting system consists of a Bruker Corporation type EQUINOX55 Fourier-transform infrared spectrometer, an aperture and an off-axis parabolic mirror. The diameter of the off-axis parabolic mirror is 80 mm with 200 mm focal length. Two apertures are used to limit stray light.

The normal spectral emissivity of sample, $_x$, is calculated by the formula,

$$x = \frac{M_x}{M_B} B$$

where $M_x$ is the radiation from the heated sample;

$M_B$ is the radiation from the blackbody at the same temperature and

$B$ is the normal spectral emissivity of the blackbody.

The emissivity at different wavelengths with a desired temperature was determined following the methods described above. The average of the emissivity for wavelength range of 5 $\mu$m-25 $\mu$m was used to represent the emissivity of different samples.

During the measurement, the wavenumber resolution of the sampling was fixed at 4 cm$^{-1}$ over the range 4000 cm$^{-1}$-400 cm$^{-1}$ (2.5 $\mu$m-25 $\mu$m), with scans repeated up to 1000 times to improve the signal-to-noise ratio. At least half an hour was needed to establish thermal equilibrium at any new temperature point for sample preparation.

As used herein, "transmissivity" refers to the degree to which a medium allows electromagnetic radiation to pass through it.

As used herein, "energy density" is calculated using the equation below:

$$M(\lambda_1 - \lambda_2, T) = \int_{\lambda1}^{\lambda2} \frac{2hc^2}{\lambda^5} \cdot \frac{1}{e^{\frac{hc}{\lambda kT}} - 1} d\lambda$$

where $\lambda_1$ and $\lambda_2$ are the wavelengths of radiation at the starting and ending point, respectively;

k is Boltzmann's constant;

t is temperature;

h is Planck's constant;

c is the speed of light in the medium, whether material or vacuum;

$M(\lambda, T)$ is the amount of energy it gives off as radiation of different wavelength; and

$M(\lambda_1 - \lambda_2, T)$ is the amount of energy the black body gives off as radiation between the wavelength range of $\lambda_1 - \lambda_2$ at the specified temperature.

The present disclosure is directed to a thermal treatment device comprising a far infrared ray material, a radiation reflection layer, and a heat energy source, wherein the radiation reflection layer is positioned between the far infrared ray material and the heat energy source.

The far infrared material is typically positioned in direct contact with the subject using the device. The far infrared material may be, for example, a fiber or fabric (e.g., a nonwoven, a woven, or combination thereof) or a film (e.g., polyethylene, polypropylene, polyethylene terephthalate, polyamide, and combinations thereof). Moreover, the far infrared ray material typically includes black carbon, ceramic powder, or mixtures thereof. Typically, black carbon or ceramic powder is impregnated into the fiber or film of the far infrared material.

Ideally, the far infrared ray material has an emissivity greater than 0.55. In one embodiment, the FIR material has an emissivity greater than 0.75.

Another essential feature of the inventive device is a radiation reflection layer, which is positioned between the far infrared material and the heat energy source. In one embodiment, the radiation reflection layer is includes a metal. Suitable metals include, for example, aluminum, copper, gold, steel, zinc, and combinations thereof. In another embodiment, the radiation reflection layer has a conductivity of at least about 10 W/mK. In addition, it is desirable that the reflection layer has a thickness from about 1 micron to about 1000 microns.

The thermal treatment device includes a heat energy source. The heat energy source may be any suitable source capable of generating or holding heat. In one embodiment, the heat energy source emits heat from about 1°C to about 10°C above the skin surface temperature of a human.

In one particular embodiment, the heat energy source comprises thermal fill materials that are a mixture of substances that chemically react exothermically. For example, several commercial hand warmers and therapeutic heat products contain an iron powder based mixture that liberates heat as the iron is oxidized upon exposure to air. These types of systems are described in detail, for example, in U.S. Patent No. 5,918,590. It is known in the art to formulate these mixtures to maintain a temperature of at least about 40°C for at least 4 hours, and up to 24 hours. Depending upon the application or desired product design, the temperature may be maintained for at least about 8 hours, at least about 10 hours, at least about 12 hours, at least about 16 hours, or whatever time period desired.

In another embodiment, the heat energy source comprises a thermal composition, which includes a microwavable heat retaining material. Suitable heat retaining materials include, for example, rice, corn, barley, cherry stones, starch-based synthetic pellets, and the like. Such materials typically retain a suitable level of heat for about 20 to about 60 minutes.

In another embodiment, the heat energy source comprises a thermal composition that includes electrically heated articles, such as a resistive heater, or a thermoelectric heating element, such as a Peltier element.

In one embodiment, the heat energy source provides heat energy at a temperature from about 38°C to about 48°C. In particular, it is desirable that the heat energy source emits heat from about 40°C to about 45°C, when the device is applied to the skin of a subject.

It is also desirable that the thermal treatment device have an energy density from about 10.83 mW/cm$^2$ to about 15.45 mW/cm$^2$ for the wavelength range of 5 to 25 $\mu$m. Correspondingly, the emissivity of the thermal treatment device should be at least about 0.75 and the surface temperature of the FIR material about 40°C to about 45°C. Preferably, it has an energy density from about 11.55 mW/cm$^2$ to about 15.45 mW/cm$^2$ for wavelength range of 5 to 25 $\mu$m which has a corresponding emissivity of the thermal treatment device of at least about 0.8 and the surface temperature of the FIR material of about 40°C to about 45°C.

In one preferred embodiment, the thermal treatment device comprises a far infrared ray material that is a nonwoven, a radiation reflection layer that is a metal, and a heat energy source, wherein the radiation reflection layer is positioned between the far infrared ray material and the heat energy source, and wherein the device has an energy density from about 10.83 mW/cm$^2$ to about 15.45 mW/cm$^2$ for wavelength range of 5 to 25 $\mu$m which means the emissivity of at least about 0.75 and the surface temperature of far infrared ray material is about 40°C to about 45°C.

The thermal treatment device may be conformed or are shaped to fit various parts of the body. For example, the thermal treatment device may be designed to the particular shape of a muscle or muscle group, such as the back, neck or shoulder of the human body.

It should be understood that the thermally conductive component can be of a various shapes, including round, semi-spherical, elongated, ellipsoidal, cylindrical, star shaped, mushroom shaped, or similar shapes. According to an embodiment of the present invention, the shapes of the thermally conductive component at the interfaces to the individual's body can be flat or non-flat, including but not limited to semi-spherical, pyramidal, conical, concave, convex, bumped, or contain an array of smaller shapes, e.g., semi-spherical protrusions.

The accompanying drawings of the present invention will now be described in detail with reference to embodiments thereof as illustrated in the accompanying drawings. However, those skilled in the art will readily appreciate that the detailed description given herein with respect to these figures is for explanatory purposes as the invention extends beyond these limited embodiments.

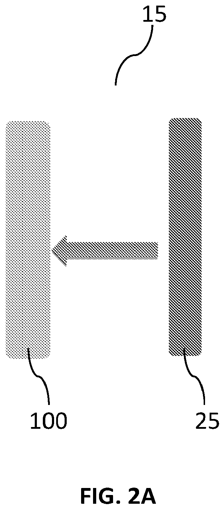

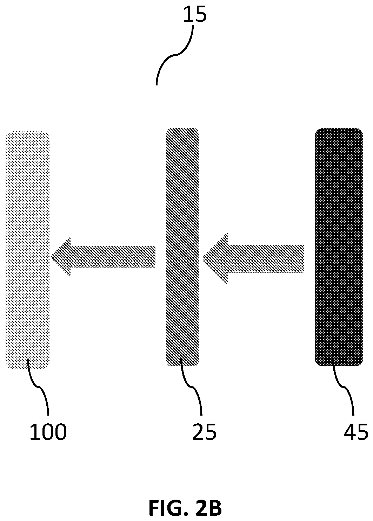

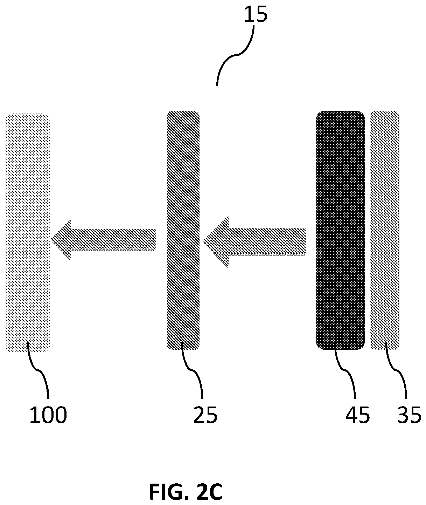

FIGS. 2A, 2B, and 2C provide examples of currently available FIR patch devices. In FIG. 2A, FIR patch 15 is essentially made of FIR material 25, which is applied to skin 100. FIG. 2B depicts another embodiment of a prior art FIR patch device 15, where an energy source 45 is added adjacent to FIR material 25. In FIG. 2C, FIR patch device 15 is shown, having an FIR material 25, an energy source 45, and a reflective layer 35, where the energy source is positioned between the FIR material and the reflective layer. In this particular configuration, the heat patch 15 will absorb all the reflection from the reflection layer 35 and transfer it into heat energy. The reflection layer 35 can only help maintain the temperature.

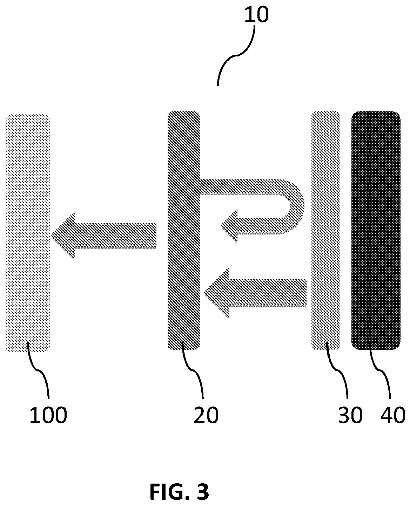

In FIG. 3, the inventive FIR heat patch device 10 is shown, having FIR material 20, reflective layer 30, and energy source 40, wherein the reflective layer is positioned between the FIR material and the energy source. The present inventors have found that by positioning the reflective layer 30 between the FIR material 20 and the energy source 40 there is a noticeable improvement in the emissivity of the device.

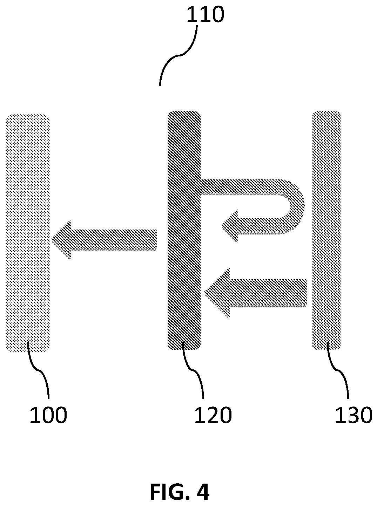

In FIG. 4, an alternative embodiment is depicted where reflective layer 130 is located adjacent to FIR material 120 in FIR patch device 110. Here again, in this particular configuration, there is a noticeable improvement in the emissivity of the device.

The present invention also includes a process for making a thermal treatment device, comprising the steps of providing a far infrared ray material and a heat energy source, and placing a radiation reflective layer between the far infrared material and the heat energy source.

The present disclosure also includes a method of treating body pain, comprising the steps of applying a thermal treatment device to a subject, wherein the device comprises far infrared ray material, a radiation reflection layer, and a heat energy source, wherein the radiation reflection layer is positioned between the far infrared ray material and the heat energy source.

The following example is provided to further illustrate the compositions and methods of the present invention. It should be understood that the present invention is not limited to the examples described.

Examples of various patch configurations were prepared and tested for Emissivity

## EXAMPLE 1

### Preparation of various reflective layer types to test Emissivity

Films produced by Clopay Plastics (Clopay FIR film D5) were used for the FIR (Far Infrared) material. Various reflective layers were combined with the Clopay FIR film D5, where the thickness of the FIR material was varied. The Emissivity of the combination was determined and presented in Table 1.

**Table 1**

|   | Reflective Layer Type | Material Thickness of FIR material ($\mu$m) | Emissivity of FIR material | Final Emissivity ($\varepsilon$) |
|---|---|---|---|---|
|   |   |   |   |   |
| 1 | Copper | 500 | 0.77 | 0.82 |
| 2 | Aluminum | 500 | 0.77 | 0.86 |
| 3 | Copper | 75 | 0.77 | 0.85 |
| 4 | Aluminum | 75 | 0.77 | 0.83 |
| *Clopay FIR film D5 is made of polyester and bamboo charcoal | | | | |

The data shows that the thickness of the FIR film layer does not substantially affect the final Emissivity.

## EXAMPLE 2

### Preparation of various reflective layer types combined with various FIR Materials

Various reflective layer types were combined with various types of film materials to determine Emissivity. The black aperture film and white films were sourced from Clopay Plastics. The data is shown in Table 2.

**Table 2**

|   | Reflective Layer Type | FIR Material Type | Emissivity of FIR material | Final Emissivity ($\varepsilon$) |
|---|---|---|---|---|
| 1 | Copper | Black Aperture Film | 0.20 | 0.40 |
| 2 | Aluminum | Black Aperture Film | 0.20 | 0.46 |
| 3 | Copper | White nonwoven film | 0.40 | 0.60 |
| 4 | Aluminum | White nonwoven film | 0.40 | 0.58 |
| 5 | Copper | White film (Clopay) | 0.25 | 0.49 |
| 6 | Aluminum | White film (Clopay) | 0.25 | 0.49 |
| 7 | Aluminum | Clopay FIR Film D5 + Aperture HTA | 0.77 | 0.74 |
| 8 | Aluminum | Clopay FIR Film D5 + HTA | 0.77 | 0.76 |

(continued)

| | Reflective Layer Type | FIR Material Type | Emissivity of FIR material | Final Emissivity ($\varepsilon$) |
|---|---|---|---|---|
| 9 | Aluminum | Clopay FIR Film D5 | 0.77 | 0.83 |

Black aperture film is Clopay's aperture FIR film (combination of polyester and bamboo charcoal) with a hole size of 100 to 200 microns and with of 26.8 gsm (gram per square meter) and thickness of 0.38 mm.
White film is Clopay's apertured film (polyester with no FIR actives) with a hole size 100 to 200 microns.
White nonwoven film is a standard wet wipe material which is made of Cellulose fibers and Polyester with 40~60 gsm and 0.25~0.4 mm thickness commercial available from Nbond Nonwovens Ltd.
HTA is a kind of nonwoven material.

The data demonstrates that different types of reflective layers can boost the Emissivity when using different types of FIR materials (e.g., films).

## EXAMPLE 3

**Preparation of various reflective layer types and various Films with Heat sources**

Various reflective layer types and various types of film materials were combined with exothermic heat patches to provide heat and FIR treatment for muscle pain relief and restoration. The data in Table 3 shows the Emissivity of the device.

Table 3

| | Heat Source (Patch) temperature (°C) | Reflective Layer Type | FIR Material Type | Emissivity of FIR material | Final Emissivity ($\varepsilon$) | Final Energy density(m W/cm$^2$) |
|---|---|---|---|---|---|---|
| 1 | >49°C | Copper | Emana[1] FIR fiber textile | 0.56 | 0.71 | >11.55 mW/cm$^2$ |
| 2 | >45°C | Copper | Clopay FIR Film D2 | 0.56 | 0.76 | >11.55 mW/cm$^2$ |
| 3 | >38°C | Copper | Clopay FIR Film D5 | 0.77 | 0.85 | >11.55 mW/cm$^2$ |
| 4 | >38°C | Gold on Copper | Clopay FIR Film D5 | 0.77 | 0.84 | >11.55 mW/cm$^2$ |
| 5 | >38°C | Steel | Clopay FIR Film D5 | 0.77 | 0.86 | >11.55 mW/cm$^2$ |
| 6 | >44°C | None | Clopay FIR Film D5 | 0.77 | 0.77 | >11.55 mW/cm$^2$ |
| 7 | >44°C | Silver (92.5%) | Clopay FIR Film D5 | 0.77 | 0.77 | >11.55 mW/cm$^2$ |
| 8 | >40°C | Stainless Steel | Clopay FIR Film D5 | 0.77 | 0.80 | >11.55 mW/cm$^2$ |
| 1: Commercially available from Emana Fiber Transforma (Emana FIR fiber textile is made of 81% Polyamide and 19% Spandex with 250 gsm)???? | | | | | | |

The data shows that different types of reflective layers can boost the Emissivity when using different types of FIR materials. The optimum ranges include a temperature above 40°C and an Emissivity above 0.8.

## Claims

**1.**    A thermal treatment device (10) comprising:

a far infrared ray material (20);
a radiation reflection layer (30); and
a heat energy source (40),
wherein the radiation reflection layer is positioned between the far infrared ray material and the heat energy source; wherein the heat energy source is configured to provide heat energy at a temperature from 38 °C to 48 °C; and wherein the far infrared material is configured to be positioned in direct contact with the subject using the device.

2. The device of claim 1, wherein the far infrared ray material comprises black carbon or ceramic powder.

3. The device of claim 1, wherein the far infrared ray material is a fiber or film.

4. The device of claim 3, wherein the fiber is selected from the group consisting of a nonwoven, a woven, and combinations thereof.

5. The device of claim 3, wherein the film is selected from the group consisting of polyethylene, polypropylene, polyethylene terephthalate, polyamide, and combinations thereof.

6. The device of claim 1, wherein the far infrared ray material has an emissivity greater than about 0.55.

7. The device of claim 1, wherein the radiation reflection layer includes a metal; optionally wherein the metal is aluminum, copper, gold, steel, zinc, and combinations thereof.

8. The device of claim 1, wherein the radiation reflection layer has a conductivity of at least 10 W/mK.

9. The device of claim 1, wherein the heat energy source emits heat from about 40°C to about 45°C, when the device is applied to the skin of a subject.

10. The device of claim 1, wherein the heat energy source is provided by a chemical reaction or by electrical energy.

11. The device of claim 1, wherein the heat energy source comprises a microwavable heat retaining material.

12. The device of claim 1, wherein the device has an emissivity of at least 0.75 at about 40°C to about 45°C.

13. The device of claim 1, wherein the far infrared ray material is a nonwoven, the radiation reflection layer is a metal; and wherein the device has an emissivity of at least 0.75 at about 40°C to about 45°C.

14. A process for making a thermal treatment device, comprising the steps of providing a far infrared ray material and a heat energy source, and placing a radiation reflective layer between the far infrared material and the heat energy source; wherein the heat energy source is configured to provide heat energy at a temperature from 38 °C to 48 °C; and wherein the far infrared material is configured to be positioned in direct contact with the subject using the device.

**Patentansprüche**

1. Wärmebehandlungsvorrichtung (10), umfassend:

ein Ferninfrarotstrahlen-Material (20);
eine strahlenreflektierende Schicht (30); und
eine Wärmeenergiequelle (40),
wobei die strahlenreflektierende Schicht zwischen dem Ferninfrarotstrahlen-Material und der Wärmeenergiequelle angeordnet ist; wobei die Wärmeenergiequelle dafür gestaltet ist, Wärmeenergie mit einer Temperatur von 38 °C bis 48 °C bereitzustellen; und wobei das Ferninfrarot-Material dafür gestaltet ist, in direktem Kontakt mit dem Subjekt, das die Vorrichtung verwendet, angeordnet zu werden.

2. Vorrichtung gemäß Anspruch 1, wobei das Ferninfrarotstrahlen-Material Ruß oder Keramikpulver umfasst.

3. Vorrichtung gemäß Anspruch 1, wobei das Ferninfrarotstrahlen-Material eine Faser oder ein Film ist.

7

4. Vorrichtung gemäß Anspruch 3, wobei die Faser ausgewählt ist aus der Gruppe bestehend aus einem Vlies, einem Gewebe und Kombinationen davon.

5. Vorrichtung gemäß Anspruch 3, wobei der Film ausgewählt ist aus der Gruppe bestehend aus Polyethylen, Polypropylen, Polyethylenterephthalat, Polyamid und Kombinationen davon.

6. Vorrichtung gemäß Anspruch 1, wobei das Ferninfrarotstrahlen-Material eine Emissivität von höher als etwa 0,55 aufweist

7. Vorrichtung gemäß Anspruch 1, wobei die strahlenreflektierende Schicht ein Metall enthält; wobei das Metall gegebenenfalls Aluminium, Kupfer, Gold, Stahl, Zink oder Kombinationen davon ist.

8. Vorrichtung gemäß Anspruch 1, wobei die strahlenreflektierende Schicht eine Leitfähigkeit von wenigstens 10 W/mK aufweist.

9. Vorrichtung gemäß Anspruch 1, wobei die Wärmeenergiequelle Wärme von etwa 40 °C bis etwa 45 °C emittiert, wenn die Vorrichtung auf die Haut eines Subjekts aufgebracht ist.

10. Vorrichtung gemäß Anspruch 1, wobei die Wärmeenergiequelle durch eine chemische Reaktion oder durch elektrische Energie bereitgestellt wird.

11. Vorrichtung gemäß Anspruch 1, wobei die Wärmeenergiequelle ein Mikrowellen-behandelbares wärmehaltendes Material umfasst.

12. Vorrichtung gemäß Anspruch 1, wobei die Vorrichtung eine Emissivität von wenigstens 0,75 bei etwa 40 °C bis etwa 45 °C aufweist.

13. Vorrichtung gemäß Anspruch 1, wobei das Ferninfrarotstrahlen-Material ein Vlies ist, die strahlenreflektierende Schicht ein Metall ist; und wobei die Vorrichtung eine Emissivität von wenigstens 0,75 bei etwa 40 °C bis etwa 45 °C aufweist.

14. Verfahren zur Herstellung einer Wärmebehandlungsvorrichtung, umfassend die Schritte des Bereitstellens eines Ferninfrarotstrahlen-Materials und einer Wärmeenergiequelle und Platzieren einer strahlenreflektierenden Schicht zwischen dem Ferninfrarotmaterial und der Wärmeenergiequelle; wobei die Wärmeenergiequelle dafür gestaltet ist, Wärmeenergie mit einer Temperatur von 38 °C bis 48°C bereitzustellen; und wobei das Ferninfrarotmaterial dafür gestaltet ist, in direktem Kontakt mit dem Subjekt, das die Vorrichtung verwendet, angeordnet zu werden.

**Revendications**

1. Dispositif de traitement thermique (10) comprenant :

   un matériau rayonnant dans l'infrarouge lointain (20) ;
   une couche de réflexion du rayonnement (30) ; et
   une source d'énergie thermique (40),
   dans lequel la couche de réflexion du rayonnement est positionnée entre le matériau rayonnant dans l'infrarouge lointain et la source d'énergie thermique, dans lequel la source d'énergie thermique est configurée pour fournir de l'énergie thermique à une température de 38 °C à 48 °C, et dans lequel le matériau infrarouge lointain est configuré pour être positionné en contact direct avec le sujet utilisant le dispositif.

2. Dispositif de la revendication 1, dans lequel le matériau rayonnant dans l'infrarouge lointain comprend du carbone noir ou de la poudre céramique.

3. Dispositif de la revendication 1, dans lequel le matériau rayonnant dans l'infrarouge lointain est une fibre ou un film.

4. Dispositif de la revendication 3, dans lequel la fibre est choisie dans le groupe constitué par un non-tissé, un tissu, et les combinaisons de ceux-ci.

**5.** Dispositif de la revendication 3, dans lequel le film est choisi dans le groupe constitué par le polyéthylène, le polypropylène, le téréphtalate de polyéthylène, le polyamide, et les combinaisons de ceux-ci.

**6.** Dispositif de la revendication 1, dans lequel le matériau rayonnant dans l'infrarouge lointain a une émissivité supérieure à environ 0,55.

**7.** Dispositif de la revendication 1, dans lequel la couche de réflexion du rayonnement comporte un métal, le métal étant éventuellement l'aluminium, le cuivre, l'or, l'acier, le zinc, ou une combinaison de ceux-ci.

**8.** Dispositif de la revendication 1, dans lequel la couche de réflexion du rayonnement a une conductivité d'au moins 10 W/mK.

**9.** Dispositif de la revendication 1, dans lequel la source d'énergie thermique émet de la chaleur d'environ 40 °C à environ 45 °C, quand le dispositif est appliqué à la peau d'un sujet.

**10.** Dispositif de la revendication 1, dans lequel la source d'énergie thermique est fournie par une réaction chimique ou par de l'énergie électrique.

**11.** Dispositif de la revendication 1, dans lequel la source d'énergie thermique comprend un matériau conservant la chaleur utilisable au micro-ondes.

**12.** Dispositif de la revendication 1, le dispositif ayant une émissivité d'au moins 0,75 à environ 40 °C à environ 45 °C.

**13.** Dispositif de la revendication 1, le matériau rayonnant dans l'infrarouge lointain étant un non-tissé, la couche de réflexion du rayonnement étant un métal, et le dispositif ayant une émissivité d'au moins 0,75 à environ 40 °C à environ 45 °C.

**14.** Procédé de fabrication d'un dispositif de traitement thermique, comprenant les étapes d'obtention d'un matériau rayonnant dans l'infrarouge lointain et d'une source d'énergie thermique, et positionnement d'une couche réfléchissant le rayonnement entre le matériau infrarouge lointain et la source d'énergie thermique, dans lequel la source d'énergie thermique est configurée pour fournir de l'énergie thermique à une température de 38 °C à 48 °C, et dans lequel le matériau infrarouge lointain est configuré pour être positionné en contact direct avec le sujet utilisant le dispositif.

**FIG. 1**

**FIG. 2A**

15

100            25            45

**FIG. 2B**

15

100    25    45  35

**FIG. 2C**

**FIG. 3**

**FIG. 4**

**EP 3 426 341 B1**

**Patent documents cited in the description**

- JP H1043319 A
- US 2012191164 A1
- EP 0963138 A2
- EP 2908807 A1
- US 2015335742 A1
- CN 1161870 A
- US 5918590 A